(19) **Europäisches Patentamt / European Patent Office / Office européen des brevets**

(11) **EP 3 775 915 B1**

(12) # EUROPEAN PATENT SPECIFICATION

(45) Date of publication and mention
of the grant of the patent:
**24.09.2025 Bulletin 2025/39**

(21) Application number: **19713499.2**

(22) Date of filing: **02.04.2019**

(51) International Patent Classification (IPC):
***G01N 33/68*** *(2006.01)*

(52) Cooperative Patent Classification (CPC):
**G01N 33/6893;** G01N 2800/18; G01N 2800/52;
G01N 2800/60

(86) International application number:
**PCT/EP2019/058213**

(87) International publication number:
**WO 2019/197200 (17.10.2019 Gazette 2019/42)**

(54) **METHODS, USES AND KITS FOR MONITORING OR PREDICTING RESPONSE TO PERIODONTAL DISEASE TREATMENT**

VERFAHREN, VERWENDUNGEN UND KITS ZUR ÜBERWACHUNG ODER VORHERSAGE DER REAKTION AUF EINE PERIODONTALE KRANKHEITSBEHANDLUNG

PROCÉDÉS, UTILISATIONS ET KITS DE SURVEILLANCE OU DE PRÉDICTION DE LA RÉPONSE À UN TRAITEMENT DE MALADIE PARODONTALE

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: **12.04.2018 EP 18166935**

(43) Date of publication of application:
**17.02.2021 Bulletin 2021/07**

(73) Proprietor: **Koninklijke Philips N.V.**
**5656 AG Eindhoven (NL)**

(72) Inventors:
• **KOOIJMAN, Gerben**
**5656 AE Eindhoven (NL)**
• **VAN HARTSKAMP, Michael Alex**
**5656 AE Eindhoven (NL)**
• **RMAILE, Amir Hussein**
**5656 AE Eindhoven (NL)**
• **GLASSE, Carl**
**5656 AE Eindhoven (NL)**
• **PRESHAW, Philip**
**5656 AE Eindhoven (NL)**
• **TAYLOR, John**
**5656 AE Eindhoven (NL)**
• **CHAPPLE, Iain Leslie Campbell**
**5656 AE Eindhoven (NL)**

• **GRANT, Melissa Mackay**
**5656 AE Eindhoven (NL)**
• **DE JAGER, Marinus Karel Johannes**
**5656 AE Eindhoven (NL)**

(74) Representative: **Philips Intellectual Property & Standards**
**High Tech Campus 52**
**5656 AG Eindhoven (NL)**

(56) References cited:
WO-A1-2016/134365     WO-A1-2016/134365
WO-A2-2014/037924

• **FUENTES L. ET AL: "Emerging horizons of salivary diagnostics for periodontal disease", BRITISH DENTAL JOURNAL : BDJ ; THE JOURNAL OF THE BRITISH DENTAL ASSOCIATION, vol. 217, no. 10, 1 November 2014 (2014-11-01), UK, pages 567 - 573, XP055896126, ISSN: 0007-0610, DOI: 10.1038/sj.bdj.2014.1005**
• **CHRISTOPH A. RAMSEIER ET AL: "Identification of Pathogen and Host-Response Markers Correlated With Periodontal Disease", JOURNAL OF PERIODONTOLOGY, vol. 80, no. 3, 1 March 2009 (2009-03-01), US, pages 436 - 446, XP055400653, ISSN: 0022-3492, DOI: 10.1902/ jop.2009.080480**

- FUENTES L. ET AL: "Emerging horizons of salivary diagnostics for periodontal disease", BRITISH DENTAL JOURNAL : BDJ ; THE JOURNAL OF THE BRITISH DENTAL ASSOCIATION, vol. 217, no. 10, 1 November 2014 (2014-11-01), UK, pages 567 - 573, XP055896126, ISSN: 0007-0610, DOI: 10.1038/sj.bdj.2014.1005
- CHRISTOPH A. RAMSEIER ET AL: "Identification of Pathogen and Host-Response Markers Correlated With Periodontal Disease", JOURNAL OF PERIODONTOLOGY, vol. 80, no. 3, 1 March 2009 (2009-03-01), US, pages 436 - 446, XP055400653, ISSN: 0022-3492, DOI: 10.1902/jop.2009.080480
- WILLIAM M. SEXTON ET AL: "Salivary biomarkers of periodontal disease in response to treatment", JOURNAL OF CLINICAL PERIODONTOLOGY, vol. 38, no. 5, 11 April 2011 (2011-04-11), DK, pages 434 - 441, XP055233059, ISSN: 0303-6979, DOI: 10.1111/j.1600-051X.2011.01706.x
- FRANCISCO ISAAC FERNANDES GOMES ET AL: "Inflammatory Cytokines Interleukin-1[beta] and Tumour Necrosis Factor-[alpha] - Novel Biomarkers for the Detection of Periodontal Diseases: a Literature Review", JOURNAL OF ORAL AND MAXILLOFACIAL RESEARCH, vol. 7, no. 2, 30 June 2016 (2016-06-30), XP055479590, DOI: 10.5037/jomr.2016.7202
- BRENDAN J. HAIGH ET AL: "Alterations in the salivary proteome associated with periodontitis", JOURNAL OF CLINICAL PERIODONTOLOGY, vol. 37, no. 3, 1 March 2010 (2010-03-01), DK, pages 241 - 247, XP055455009, ISSN: 0303-6979, DOI: 10.1111/j.1600-051X.2009.01525.x
- GHALLAB NOHA AYMAN: "Diagnostic potential and future directions of biomarkers in gingival crevicular fluid and saliva of periodontal diseases: Review of the current evidence", ARCHIVES OF ORAL BIOLOGY, vol. 87, March 2018 (2018-03-01), pages 115 - 124, XP085348826, ISSN: 0003-9969, DOI: 10.1016/J.ARCHORALBIO.2017.12.022
- HÉLÈNE RANGÉ ET AL: "Orosomucoid, a New Biomarker in the Association between Obesity and Periodontitis", PLOS ONE, vol. 8, no. 3, 1 March 2013 (2013-03-01), US, pages e57645 - 1, XP055478406, ISSN: 1932-6203, DOI: 10.1371/journal.pone.0057645
- JANSKY L ET AL: "Immune system of cold-exposed and cold-adapted humans", EUROPEAN JOURNAL OF APPLIED PHYSIOLOGY AND OCCUPATIONAL PHYSIOLOGY, vol. 72, no. 5-6, 1996, pages 445 - 450, XP009505733, ISSN: 0301-5548
- MCKINDLEY DAVID S ET AL: "Effect of acute phase response on phenytoin metabolism in neurotrauma patients", JOURNAL OF CLINICAL PHARMACOLOGY, vol. 37, no. 2, 1997, pages 129 - 139, XP009505734, ISSN: 0091-2700

**Description**

FIELD OF THE INVENTION

The invention is in the field of oral care, and pertains to saliva-based evaluation of response to treatment for periodontal disease. Particularly, the invention pertains to a kit, use and methods for assessing or predicting the response to treatment of a patient suffering from periodontal disease.

BACKGROUND OF THE INVENTION

Gum inflammation, or gingivitis, is a non-destructive periodontal disease caused mainly by the adherence of dental bacterial biofilms, or dental plaque, to the tooth surface. If not detected and treated, the reversible gingivitis usually leads to the inflammation of the tissues surrounding the tooth (i.e. periodontal tissues), a condition defined as periodontitis, which is irreversible and causes tissue destruction and alveolar bone loss, and ultimately results in the loss of teeth. During the progression of gum disease, there are usually clinical signs and symptoms associated with it, such as the swelling of the gums, the change in color from pink to dark red, the bleeding of the gums, bad breath, and the gums becoming more tender or painful to touch.

Periodontitis is a chronic multifactorial inflammatory disease caused by oral microorganisms and characterized by progressive destruction of the hard (bone) and soft (periodontal ligament) tissues, ultimately leading to tooth mobility and loss. This is to be distinguished from gingivitis which is a reversible infection and inflammation of the gum tissues. Inflammatory periodontitis is one of the most prevalent chronic human diseases and a major cause of adult tooth loss. In addition to the substantial negative impact of periodontitis on oral health, there is also mounting evidence that periodontitis has systemic consequences and that it is a risk factor for several systemic diseases, including heart diseases (e.g. atherosclerosis, stroke), diabetes, pregnancy complications, rheumatoid arthritis and respiratory infections.

Early and accurate diagnosis of periodontal disease, thus, is important from both an oral and overall health perspective. Furthermore, it would be desirable to be able to determine accurately whether a treatment of periodontal disease is, or is likely to be, effective in a patient. In particular, it would be desirable to be able to predict whether a treatment for periodontal disease is likely to be effective before it is provided to the patient or at an early stage of treatment.

Periodontal diseases are still poorly diagnosed in general dental practice, resulting in relatively low rates of therapeutic intervention and significant amounts of untreated cases. Current diagnosis relies on imprecise, subjective clinical examination of oral tissue condition (color, swelling, extent of bleeding on probing, probing pocket depth; and bone loss from oral x-rays) by dental professionals. These conventional methods are time consuming, and some of the techniques used (pocket-depth, x-ray) reflect historic events, such as past disease activity, rather than current disease activity or susceptibility to further disease.

Similarly, for periodontal patients receiving treatment, the response to treatment currently has to be clinically assessed via these conventional methods post-treatment, resulting in high cost and potentially inaccurate monitoring of the patient's condition. Furthermore, the possibility of predicting treatment response can be of large value, as treatment strategy may be adopted accordingly. Thereby it is desirable to measure current disease activity, a subject's susceptibility to further periodontal disease, and what treatment is likely to be successful in that patient. Hence, more objective, faster, accurate, easier-to-use diagnostics - ideally with predictive value - and which preferably may also be performed by non-specialists, are desirable.

Saliva or oral fluids have long been advocated as a diagnostic fluid for oral and general diseases, and with the advent of miniaturized biosensors, also referred to as lab-on-a-chip, point of care diagnostics for rapid chair-side testing have gained greater scientific and clinical interest. Especially for periodontal disease detection, inflammatory biomarkers associated with tissue inflammation and breakdown may easily end up in saliva due to proximity, suggesting saliva has strong potential for periodontal disease detection. Indeed, this area thus has gained significant interest and encouraging results have been presented. For example, Ramseier et al (J Periodontol. 2009 Mar;80(3):436-46) identified host- and bacterially derived biomarkers correlated with periodontal disease. However, no definite test has emerged yet.

Biomarkers represent biological indicators that underpin clinical manifestations, and as such are objective measures by which to diagnose clinical outcomes of periodontal disease. Ultimately, proven biomarkers could be utilized to assess risk for future disease, to identify disease at the very earliest stages, to identify response to initial therapy, and to allow implementation of preventive strategies.

Previous limitations to the development of point-of-care tests for salivary biomarkers included a lack of technologies that were adaptable to chair-side applications and an inability to analyze multiple biomarkers in individual samples. Also the selection of which multiple biomarkers to include in such a test has not been adequately addressed in the literature nor implemented in practical tests.

Moreover, periodontitis can manifest itself across the entire spectrum of severity ranging from mild to advanced forms of the disease. To assess the severity of the condition easily, dentists often classify patients suffering from periodontitis into two groups - those suffering from *mild* periodontitis, and those suffering from *advanced* periodontitis. The available methods of making such an assessment, however, involve a labor intensive process that a dentist will not perform routinely on every patient and/or on every visit, and that is impossible to perform by a consumer (self-diagnosis).

A document 'Salivary biomarkers of periodontal disease in response to treatment' by W.M Sexton et. al. published in Journal of Clinical Periodontology 2011; 38: 434-441 describes longitudinal assessment of salivary biomarkers of periodontitis to determine their response to therapy.

It would be desired to provide a simpler process, and particularly a process that requires only that a small saliva sample is taken from a patient, and possibly by the patient him- or herself. It is desired that such a sample be entered into an *in vitro* diagnostic device, which will allow, based on measurement, a classification of the saliva sample such that it can return an indication of the likelihood that periodontal disease in the patient is being, or is likely to be, effectively treated.

SUMMARY OF THE INVENTION

To better address the foregoing desires, the invention, in one aspect, concerns an *in vitro* method as claimed in claim 1.

In another aspect, the invention presents the use as claimed in claim 5.

Optionally, the age of the patient is also used as a biomarker.

In a further aspect, the invention resides in a system as claimed in claim 7.

The system optionally contains a data connection to an interface, particularly a graphical user interface, capable of presenting information, preferably also capable of putting in information, said interface being either a part of the system or a remote interface.

Optionally one or more of the foregoing items, particularly the processor, are enabled to function "in the cloud", i.e., not on a fixed machine, but by means of an internet-based application.

In a still further aspect, the invention provides a kit as claimed in claim 11.

In yet another aspect, the invention provides an *in vitro* method as claimed in claim 14.

In a further aspect, the invention provides a method as claimed in claim 15.

BRIEF DESCRIPTION OF THE DRAWINGS

Fig. 1 schematically represents a system for use in the method as described in this disclosure.
Fig. 2 displays the number of identified biomarker protein panels having at most 4 protein markers as function of the threshold in classification performance in terms of Receiver-Operator-Characteristic Area-Under-the-Curve when leave-one-out cross validation is employed (ROC AUC LOOCV), for assessing treatment response. Separate graphs are shown for exclusion of age as predictor as well as inclusion of age.

DETAILED DESCRIPTION OF THE INVENTION

In a general sense, the invention is based on the judicious insight that certain combinations of protein biomarkers in a sample of saliva of a human patient, can be used for assessing or predicting the response to treatment of periodontal disease in a patient. The biomarker combinations in saliva are able to differentiate a successful response to periodontitis treatment from an unsuccessful response to periodontitis treatment. This insight is based at least in part on the finding that the usefulness of a biomarker for <u>diagnosing</u> periodontal disease (e.g. before treatment) does not necessarily mean that it is also useful for monitoring the treatment of that periodontal disease. The current disclosure presents clinical definitions of various levels of treatment response, and identifies the salivary protein marker combinations that enable assessment or prediction of treatment response from measurement of their concentrations post-treatment, or (for prediction) pre-treatment.

The biomarker proteins are Alpha-1-acid glycoprotein (A1AGP), Interleukin-1-beta (IL-1β), Matrix metalloproteinase-8 (MMP-8), Matrix metalloproteinase-9 (MMP-9), Keratin-4 (K-4), Hepatocyte Growth Factor (HGF), Haemoglobin-beta (Hb-β), S100 calcium binding protein A9 (S100A9) and Free Light Chain-kappa (FLC κ). The following combination of these proteins are used to monitor or predict the treatment of periodontal disease according to the invention:

Alpha-1-acid glycoprotein (A1AGP) and Interleukin 1-β (IL-1β) and at least one of Matrix metalloproteinase-9 (MMP9), Matrix metalloproteinase-8 (MMP8); Matrix metalloproteinase-9 (MMP9); and Keratin 4; or
Alpha-1-acid glycoprotein (A1AGP) in combination with at least one of the proteins Matrix metalloproteinase-8 (MMP8) and Interleukin 1-β (IL-1β); or

Alpha-1-acid glycoprotein (A1AGP) and Matrix metalloproteinase-9 (MMP9)

The subject's age may optionally be included as an additional marker.

In one embodiment, the method assesses the response of a human patient previously diagnosed as having periodontitis and that has received treatment for that periodontitis.

In another embodiment, the method predicts the response of a human patient to a treatment for periodontitis. This may be when the treatment has not yet been administered to the patient. Alternatively, a treatment may have been administered recently to the patient and it is desired to know at an early stage whether it is likely to be effective. Typically, the treatment is first administered at least about one week, about two weeks or about three weeks, for example at least about 7 days, at least about 14 days or at least about 21 days prior to assessment of the patient using the method of the invention. The treatment may begin between about one week and between about one month prior to assessment. The patient may optionally be assessed at two, three or four week intervals after first treatment, for example at weeks 3, 6 and 9 after treatment, or weeks 4, 8 and 12 after treatment. In this predictive embodiment, the concentrations of the proteins MMP8, IL1B and A1AGP may be detected; Pyruvate Kinase may also be included as an additional protein biomarker in this panel.

Alpha-1-acid glycoprotein (A1AGP) is a plasma alpha-globulin glycoprotein synthesized primarily by the liver. It is also sometimes known as Orosomucoid. It functions as a transport protein in the blood acts as a carrier of basic and neutrally charged lipohillic compounds. It is also believed to regulate the interaction between blood cells and endothelial cells.

IL-1$\beta$ is a member of the interleukin 1 family of cytokines. This cytokine is produced by activated macrophages as a proprotein, which is proteolytically processed to its active form by caspase 1 (CASP1/ICE). This cytokine is an important mediator of the inflammatory response, and is involved in a variety of cellular activities, including cell proliferation, differentiation, and apoptosis.

MMPs are a family of enzymes that are responsible for the degradation of extracellular matrix components such as collagen, proteoglycans, laminin, elastin, and fibronectin. They play a central role in the periodontal ligament (PDL) remodelling, both in physiological and pathological conditions. MMP-8, also known as neutrophil collagenase or PMNL collagenase (MNL-CL), is a collagen protease enzyme which is present in the connective tissue of most mammals. MMP-9, also known as 92 kDa type IV collagenase, 92 kDa gelatinase or gelatinase B (GELB), is a matrixin, a class of enzymes that belong to the zinc-metalloproteinases family involved in the degradation of the extracellular matrix.

Keratin-4 (K4), also known as cytoskeletal Keratin 4 (CYK4) or cytokeratin-4 (CK-4) is a protein that in humans is encoded by the KRT4 gene. It is a member of the keratin gene family. The type II cytokeratins consist of basic or neutral proteins which are arranged in pairs of heterotypic keratin chains coexpressed during differentiation of simple and stratified epithelial tissues. The type II cytokeratin CK4 is specifically expressed in differentiated layers of the mucosal and esophageal epithelia with family member KRT13. Mutations in these genes have been associated with White Sponge Nevus, characterized by oral, esophageal, and anal leukoplakia. The type II cytokeratins are clustered in a region of chromosome 12q12-q13.

Hepatocyte Growth Factor (HGF) is a paracrine cellular growth, motility and morphogenic factor. It is secreted by mesenchymal cells and targets and acts primarily upon epithelial cells and endothelial cells, but also acts on haemopoietic progenitor cells. HGF has been shown to have a major role in myogenesis and in wound healing. Its ability to stimulate mitogenesis, cell motility, and matrix invasion gives it a central role in angiogenesis, tumorogenesis, and tissue regeneration. HGF stimulates growth of epithelial cells and prevents regeneration of the connective tissue attachment. HGF is known as a serum marker indicating disease activity in various diseases.

Haemoglobin (Hb) is the iron-containing oxygen-transport metalloprotein in the red blood cells of nearly all vertebrates as well as the tissues of some invertebrates. Haemoglobin-beta (also known as beta globin, HBB, $\beta$-globin, and haemoglobin subunit beta) is a globin protein, which along with alpha globin (HBA), makes up the most common form of haemoglobin in adult humans, the HbA. Hb-$\beta$ is typically 146 amino acids long and has a molecular weight of 15,867 Da. Normal adult human HbA is a heterotetramer consisting of two alpha chains and two beta chains. Hb-$\beta$ is encoded by the HBB gene on human chromosome 11.

S100 calcium binding protein A9 (S100A9), also known as calgranulin B, is a calcium- and zinc-binding protein which plays a prominent role in the regulation of inflammatory processes and immune response. It can induce neutrophil chemotaxis, adhesion, can increase the bactericidal activity of neutrophils by promoting phagocytosis via activation of SYK, PI3K/AKT, and ERK1/2 and can induce degranulation of neutrophils by a MAPK-dependent mechanism.

Free Light Chain proteins are immunoglobulin light chains. They are not associated with an immunoglobulin heavy chain. Unlike a typical whole immunoglobulin molecule, a Free Light Chain protein is not covalently linked to an immunoglobulin heavy chain, e.g. the Free Light Chain is not disulphide bonded to a heavy chain. Typically the Free Light Chain comprises approximately 220 amino acids. Typically, the Free Light Chain protein comprises a variable region (often referred to as the Light Chain variable Region, $V_L$) and a constant region (often referred to as the Light Chain constant Region, $C_L$). Humans produce two types of immunoglobulin light chains, named with the letter kappa ($\kappa$) and lambda ($\lambda$). Each of these can be further divided into sub-groups based on variation in the variable region, with four kappa subtypes (Vκ1, Vκ2, Vκ3 and Vκ4) and six lambda subtypes (Vλ1, Vλ2, Vλ3, Vλ4, Vλ5 and Vλ6). Free Light Chain κ is typically monomeric. Free Light Chain λ is typically dimeric, linked by disulphide bonding (to another Free Light Chain λ). Polymeric forms of Free Light Chain λ and of Free Light Chain κ have been identified. Free light chains are produced by bone marrow and lymph node cells as well as locally in the periodontium by diffuse lymphocytes, and are rapidly cleared from the blood and catabolised by the kidneys. Monomeric free light chains are cleared in 2-4 hours, and dimeric free light chains in 3-6 hours.

The proteins mentioned above are known in the art. The skilled person is aware of their structure, and of methods to detect them in an aqueous sample, such as a saliva sample. Hereinafter the following protein biomarker combinations are collectively referred to as "the biomarker panels of the invention":

Alpha-1-acid glycoprotein (A1AGP) and Interleukin 1-β (IL-1β) and
at least one of Matrix metalloproteinase-9 (MMP9), Matrix metalloproteinase-8 (MMP8) and Keratin 4;
Alpha-1-acid glycoprotein (A1AGP) and Matrix metalloproteinase-9 (MMP9);
Alpha-1-acid glycoprotein (A1AGP) in combination with at least one of the proteins Matrix metalloproteinase-8 (MMP8); and Interleukin 1-β (IL-1β.

A biomarker panel of the invention, in one embodiment, may consist of the protein biomarkers identified. Preferably, a biomarker panel of the invention consists of not more than four of the protein biomarkers identified in the invention. In addition to the biomarker panels of the invention, other biomarkers and or data, such as demographic data (e.g., age, sex) can be included in a set of data applied for the determination of the type of periodontitis. An example of an additional protein biomarker is pyruvate kinase. An example of an extended biomarker panel of the invention is MMP8, IL-1β, A1AGP, and Pyruvate kinase.

When other biomarkers are optionally included, the total number of biomarkers (i.e. the biomarker panel of the invention plus other biomarkers) is typically 4, 5 or 6.

However, a desirable advantage of the present invention is that the classification of periodontal disease in a patient can be determined by measuring preferably not more than four biomarkers, and more preferably measuring only three biomarkers, with the biomarker panel consisting of Alpha-1-acid glycoprotein (A1AGP), Interleukin 1-β (IL-1β) and at least one of Matrix metalloproteinase-9 (MMP9), Matrix metalloproteinase-8 (MMP8) and Keratin-4 being preferred. Particularly, the determination does not need to involve the use of other data, which advantageously provides a simple and straightforward diagnostic test. The biomarker panels identified herein allow the detection of a successful outcome of periodontitis treatment.

The method, as desired, requires only that a small saliva sample, e.g. a dropsize, is taken from the subject. The size of the sample will typically range of from 0.1 μl to 2 ml, such as 1-2 ml, whereby smaller amounts, e.g., 0. 1 to 100 μl can be used for *in vitro* device processing, and whereby taking a larger sample, such as up to 20 ml, such as 7.5 to 17 ml, is also possible.

This sample is entered into an *in vitro* diagnostic device, which measures the concentrations of the proteins involved, and which returns an outcome, classifying the subject on the basis of a likelihood of successful periodontal disease treatment.

The ease of use of this invention will make it possible to test the majority of dental patients having periodontal disease, on a regular basis (e.g. as part of a regular dental check or even at home). This allows, *inter alia*, detecting whether treatment of periodontitis is or will be successful, and thus enables a more informed approach to treatment of the periodontitis, whereby a successful treatment can be continued and an unsuccessful treatment ceased or not begun. The ability to assess that the therapy is successful is beneficial to confirm, for example, that the patient's current treatment is satisfactory. Particularly, the method is also suitable for self-diagnosis, whereby the steps of taking the sample and entering it into a device can be conducted by the patient him- or herself.

The patient may typically be known to have periodontal disease when the invention is carried out. The patient may typically be known to have periodontitis when the invention is carried out. The periodontitis may be mild or advanced. In certain embodiments therefore, the method is for assessing whether a human patient, known to have periodontitis, is being (or will be) successfully treated for that condition.

The therapy that is assessed as successful or not may be any therapy for periodontal disease. Therapeutic agents and dental procedures, or a combination of therapeutic agents and dental procedures, are known and may be used. Known therapeutic agents include the administration of antimicrobial-containing agents such as a mouthwash, chip, gel or microsphere. A typical antimicrobial agent for use in treating gingivitis and periodontitis is chlorhexidine. Other therapeutic agents include antibiotics, typically orally-administered antibiotics, and enzyme suppressants such as doxycycline. Known non-surgical therapeutic procedures include: root surface instrumentation to disrupt subgingival plaque biofilms; scaling and root planing (SRP); and interproximal cleaning. Known surgical procedures include surgical pocket reduction, flap surgery, gum grafts or bone grafts. The therapy will preferably be a therapeutic agent such as an anti-microbial agent, typically an anti-microbial mouthwash.

The need for, and number of, optional visits to a dentist or hygienist for therapy can vary, according to clinical need and patient preference. An initial phase of clinical treatment is typically completed in a fewer number of longer appointments. In this embodiment, patients may attend a dental clinic for 2 appointments of approximately 1-2 hours each for the initial therapy. Following this, frequent recall within the early post-treatment period can check on healing, motivate patients, reinforce oral hygiene, and provide prophylaxis for removal of reforming plaque. This is typically achieved with short (e.g. 15-30 min) appointments at 2-4 week intervals for the next 1-2 months after the initial treatment period.

Accordingly, in certain embodiments the treatment phase may include 1-2 clinical appointments for root surface instrumentation. Following this, patients may return to the dentist or hygienist approximately 3, 6 and 9 weeks later for recall appointments (prophylaxis, motivation, reinforcement of oral hygiene). Within this schedule, treatment protocols can be implemented according to the clinical needs of the individual.

In certain embodiments, the assessment or prediction method of the invention may be carried out at any clinical visit subsequent to the initial therapy.

A method of the invention typically comprises detecting the aforementioned at least two proteins making up a biomarker panel of the invention, and optional further biomarker proteins, by using one or more detection reagents.

Typically, a treatment can be considered successful when inflammation levels are significantly lowered, whereas pocket depth may remain relatively high (i.e. compared to healthy individuals or gingivitis patients). Therefore, application of a disease classification (e.g. discriminating between health, gingivitis, mild periodontitis, and advanced periodontitis) based on salivary protein markers of disease *per se*, to patients post-treatment may not be suitable to assess treatment response. The biomarker proteins of the invention overcome this problem and are able to determine the response to treatment, specifically.

The "saliva" that is tested according to the invention may be undiluted saliva, which may be obtained by spitting or swabbing, or diluted saliva, which may be obtained by rinsing the mouth with a fluid. Diluted saliva may be obtained by the patient rinsing or swilling their mouth for a few seconds with sterile water (for example 5ml or 10ml) or other suitable fluid, and spitting into a container. Diluted saliva may sometimes be referred to as an oral rinse fluid.

By "detecting" is meant measuring, quantifying, scoring, or assaying the concentration of the biomarker proteins. Methods of evaluating biological compounds, including biomarker proteins, are known in the art. It is recognized that methods of detecting a protein biomarker include direct measurements and indirect measurements. One skilled in the art will be able to select an appropriate method of assaying a particular biomarker protein.

The term "concentration" with respect to the protein biomarkers is to be given its usual meaning, namely the abundance of the protein in a volume. Protein concentration is typically measured in mass per volume, most typically mg/ml or $\mu$g/ml, but sometimes as low as pg/ml. An alternative measure is Molarity (or Molar concentration), mol/L or "M". The concentration can be determined by detecting the amount of protein in a sample of known, determined or pre-determined volume.

An alternative to determining the concentration is to determine the absolute amount of the protein biomarker in the sample, or determining the mass-fraction of the biomarker in the sample, for example the amount of the biomarker relative to the total of all other proteins in the sample.

A "detection reagent" is an agent or compound that specifically (or selectively) binds to, interacts with or detects the protein biomarker of interest. Such detection reagents may include, but are not limited to, an antibody, polyclonal antibody, or monoclonal antibody that preferentially binds the protein biomarker.

The term "periodontal disease" refers to gingivitis and periodontitis (mild and advanced). A patient that is free from periodontal disease is said to be healthy.

The phrase "specifically (or selectively) binds" or "specifically (or selectively) immunoreactive with", when referring to a detection reagent, refers to a binding reaction that is determinative of the presence of the protein biomarker in a heterogeneous population of proteins and other biologics. Thus, under designated immunoassay conditions, the specified detection reagent (e.g. antibody) binds to a particular protein at least two times the background and does not substantially bind in a significant amount to other proteins present in the sample. Specific binding under such conditions may require an antibody that is selected for its specificity for a particular protein. A variety of immunoassay formats may be used to select antibodies specifically immunoreactive with a particular protein. For example, solid-phase ELISA immunoassays (enzyme linked immunosorbent assay) are routinely used to select antibodies specifically immunoreactive with a protein (*see, e.g.*, Harlow & Lane, Antibodies, A Laboratory Manual (1988), for a description of immunoassay formats and conditions that can be used to determine specific immunoreactivity). Typically a specific or selective reaction will be at least twice the background signal or noise and more typically more than 10 to 100 times the background.

"Antibody" refers to a polypeptide ligand substantially encoded by an immunoglobulin gene or immunoglobulin genes, or fragments thereof, which specifically binds and recognizes an epitope (e.g., an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, e.g., as intact immunoglobulins or as a number of well characterized fragments produced by digestion with various peptidases.

This includes, e.g., Fab' and F(ab)'2 fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH1, CH2 and CH3, but does not include the heavy chain variable region. The antibody may be a bispecific antibody, e.g. an antibody that has a first variable region that specifically binds to a first antigen and a second variable region that specifically binds to a second, different, antigen. Use of at least one bispecific antibody can reduce the number of detection reagents needed.

Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. Specificity may also be referred to as the true negative rate.

The biomarker protein(s) of the invention can be detected in a sample by any means. Preferred methods for biomarker detection are antibody-based assays, protein array assays, mass spectrometry (MS) based assays, and (near) infrared spectroscopy based assays. For example, immunoassays, include but are not limited to competitive and non-competitive assay systems using techniques such as Western blots, radioimmunoassays, ELISA, "sandwich" immunoassays, immunoprecipitation assays, precipitin reactions, gel diffusion precipitin reactions, immunodiffusion assays, fluorescent immunoassays and the like. Such assays are routine and well known in the art. Exemplary immunoassays are described briefly below (but are not intended by way of limitation).

Immunoprecipitation protocols generally comprise lysing a population of cells in a lysis buffer such as RIPA buffer (1% NP-40 or Triton X-100, 1 % sodium deoxycholate, 0.1% SDS, 0.15 M NaC1, 0.01 M sodium phosphate at pH 7.2, 1 % Trasylol) supplemented with protein phosphatase and/or protease inhibitors (e.g., EDTA, PMSF, aprotinin, sodium vanadate), adding an antibody of interest to the cell lysate, incubating for a period of time (e.g., 1-4 hours) at 4°C, adding protein A and/or protein G sepharose beads to the cell lysate, incubating for about an hour or more at 4°C, washing the beads in lysis buffer and resuspending the beads in SDS/sample buffer. The ability of the antibody to immunoprecipitate a particular antigen can be assessed by, e.g., western blot analysis. One of skill in the art would be knowledgeable as to the parameters that can be modified to increase the binding of the antibody to an antigen and decrease the background (e.g., pre-clearing the cell lysate with Sepharose beads).

Western blot analysis generally comprises preparing protein samples, electrophoresis of the protein samples in a polyacrylamide gel (e.g., 8%-20% SDS-PAGE depending on the molecular weight of the antigen), transferring the protein sample from the polyacrylamide gel to a membrane such as nitrocellulose, PVDF or nylon, blocking the membrane in blocking solution (e.g., PBS with 3% BSA or non-fat milk), washing the membrane in washing buffer (e.g., PBS-Tween 20), blocking the membrane with primary antibody (the antibody of interest) diluted in blocking buffer, washing the membrane in washing buffer, blocking the membrane with a secondary antibody (which recognizes the primary antibody, e.g., an anti-human antibody) conjugated to an enzymatic substrate (e.g., horseradish peroxidase or alkaline phosphatase) or radioactive molecule (e.g., 32P or 125I) diluted in blocking buffer, washing the membrane in wash buffer, and detecting the presence of the antigen. One of skill in the art would be knowledgeable as to the parameters that can be modified to increase the signal detected and to reduce the background noise.

ELISAs typically comprise preparing antigen (i.e. the biomarker protein of interest or fragment thereof), coating the well of a 96- well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art.

Since multiple markers are used, a threshold can be determined on the basis of the joint concentrations of these biomarkers. This threshold determines whether a patient is classified as having been successfully treated or not. The invention reflects the insight that successful response to treatment of periodontitis can be distinguished from unsuccessful response to treatment of periodontitis with sufficient accuracy based on a measurement of the combination of biomarkers as indicated above.

This insight supports another aspect, the invention, which is the use of the protein combinations of the invention, as biomarkers in a saliva sample of a human patient, for assessing whether a periodontitis treatment is or will be successful in the patient. For the avoidance of doubt, the protein combinations of this aspect are:

(i) Alpha-1-acid glycoprotein (A1AGP) and at least one of Interleukin-1-beta (IL-1β) and Matrix metalloproteinase-8 (MMP-8); or

(ii) Alpha-1-acid glycoprotein (A1AGP) and Interleukin-1-beta (IL-1β), and at least one of Matrix metalloproteinase-8 (MMP-8), Matrix metalloproteinase-9 (MMP-9) and Keratin-4 (K-4); or

(iii) Matrix metalloproteinase-9 (MMP-9) and Alpha-1-acid glycoprotein (A1AGP).

This use can be implemented in a method as substantially described hereinbefore and hereinafter.

The method of the invention comprises determining at least one testing value reflecting the joint concentrations measured for said proteins. A joint concentration value can be any value obtained by input of the concentrations as determined and an arithmetic operation of these values. This can, e.g., be a simple addition of the concentrations. It can also involve multiplying each concentration with a factor reflecting a desired weight of these concentrations, and then adding up the results. It can also involve multiplying the concentrations with each other, or any combination of multiplication, division, subtraction, exponentiation, and addition. It can further involve raising concentrations to some power. Optionally, the testing value reflects the joint concentrations determined for said proteins in combination with the age of the subject.

The resulting joint concentration value may be compared with one or more threshold values reflecting in the same manner the joint concentrations associated with the successful treatment of periodontitis. The comparison allows assessing whether the testing value is indicative of the presence of successful treatment in the patients whose saliva is subjected to the test.

The threshold value can, e.g., be the joint concentration value, obtained in the same manner on the basis of the concentrations determined for the same proteins in a reference sample associated with the successful treatment of periodontitis, e.g. in a patient that was previously diagnosed with periodontitis and has been treated to reduce the severity of the disease. Typically, thereby a value reflecting the same or higher joint concentration is indicative of the successful treatment of periodontitis in a tested patient. Analogously, a value reflecting a lower joint concentration in the saliva of a tested periodontitis patient, indicates that the periodontitis has not been successfully treated. However, it will be understood that it is also possible to calculate a threshold value (e.g. by using a negative multiplier) such that a testing value indicating successful; treatment would be below the threshold, and a testing value indicating unsuccessful treatment, would be above the threshold.

The threshold value can also be determined on the basis of measuring the concentrations of the present biomarker proteins in a set of samples, including patients known to have been successfully treated and patients that were not successfully treated. Thereby the measured concentration values can be subjected to statistical analysis, possibly including machine learning methods, allowing to discriminate, with the desired sensitivity and specificity, patients classified as having been successfully treated or not. Therefrom, the desired threshold value(s) can be obtained. On the basis of this threshold value, a sample to be tested can be subjected to the same concentration measurement, and the concentration values are then processed, in the same manner in which the threshold value(s) is obtained, so as to determine a joint concentration value that can be compared with the threshold, thus allowing the tested sample to be classified as "yes" or "no" for successful treatment.

In an interesting embodiment, the joint concentration value is obtained in the form of a score as follows. A numerical value (protein concentration values in e.g. ng/ml) is assigned to each measurement, and these values are used in a linear or non-linear combination to calculate a score between zero and one. In the event that the threshold value is determined on the basis of a set of subjects as mentioned above, the score between 0 and 1 is typically calculated with the sigmoid function that takes the joint concentration as input (as shown further on).

When the score exceeds a certain threshold, the method indicates successful treatment of periodontitis. The threshold may be chosen based on the desired sensitivity and specificity.

Clinical definitions as acknowledged in the art are based on the following:

*Gingival Index (GI)*

A full mouth gingival index will be recorded based on the Lobene Modified Gingival Index (MGI) rated on a scale of 0 to 4, where:

- 0 = absence of inflammation,
- 1 = mild inflammation; slight change in color little change in texture of any portion of but not the entire margin or papillary gingival unit,
- 2 = mild inflammation; but involving entire margin or papillary unit,
- 3 = moderate inflammation; glazing, redness, oedema and/or hypertrophy of margin or papillary unit,
- 4 = severe inflammation; marked redness, oedema and/or hypertrophy of marginal or papillary gingival unit, spontaneous bleeding, congestion, or ulceration].

*Probing depths (PD)*

Probing depths will be recorded to the nearest mm using a manual UNC-15 periodontal probe. Probing depth is the distance from the probe tip (assumed to be at the base of the pocket) to the free gingival margin.

*Gingival recession (REC)*

Gingival recession will be recorded to the nearest mm using a manual UNC-15 periodontal probe. Gingival recession is the distance from the free gingival margin to the cemento-enamel junction. Gingival recession will be indicated as a positive number and gingival overgrowth will be indicated as a negative number.

*Clinical attachment loss (CAL)*

Clinical attachment loss will be calculated as the sum of probing depth + recession at each site.

*Bleeding on probing (BOP)*

Following probing, each site will be assessed for bleeding on probing, if bleeding occurs within 30s of probing, a score of 1 will be assigned for the site, otherwise a score of 0 will be assigned.

The resulting subject group (patient group) definition is as follows:

- Healthy group (H): PD $\leq$ 3 mm in all sites (but would allow up to four 4 mm pockets at distal of last standing molars), no sites with interproximal attachment loss, GI of $\geq$ 2.0 in $\leq$ 10% sites, %BOP scores $\leq$ 10%;
- Gingivitis group (G): GI $\geq$ 3.0 in > 30% of sites, no sites with interproximal attachment loss, no sites with PD > 4 mm, % BOP scores > 10%;
- Mild-moderate periodontitis group (MP): interproximal PD of 5-7 mm, (equating to approximately 2-4 mm CAL) at $\geq$ 8 teeth, %BOP scores > 30%;
- Advanced periodontitis group (AP): interproximal PD of $\geq$ 7 mm, (equating to approximately $\geq$ 5 mm CAL) at $\geq$ 12 teeth, %BOP scores > 30%.

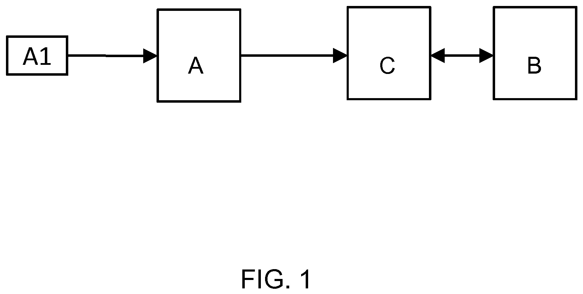

In an embodiment, the method of the invention makes use of a system as represented schematically in Fig. 1. The system can be a single apparatus having various device components (units) integrated therein. The system can also have its various components, or some of these components, as separate apparatuses. The components shown in Fig. 1 are a measurement device (A), a graphical user interface (B) and a computer processing unit (C).

As mentioned above, the system of the invention comprises a data connection to an interface, whereby the interface itself can be a part of the system or can be a remote interface. The latter refers to the possibility to use a different apparatus, preferably a handheld apparatus such as a smartphone or a tablet computer, for providing the actual interface. The data connection in such cases will preferably involve wireless data transfer such as by Wi-Fi or Bluetooth, or by other techniques or standards.

The measurement device (A) is configured to receive a saliva sample, for example by putting a drop of saliva on a cartridge (A1), which can be inserted into the device (A). The device can be an existing device that is capable to determine, from the same saliva sample, the concentrations of at least a biomarker protein combination of the invention, i.e.:

Alpha-1-acid glycoprotein (A1AGP) and Interleukin 1-$\beta$ (IL-1$\beta$) and
at least one of Matrix metalloproteinase-9 (MMP9), Matrix metalloproteinase-8 (MMP8) and Keratin 4;
Alpha-1-acid glycoprotein (A1AGP) in combination with at least one of the proteins Matrix metalloproteinase-8 (MMP8) or Interleukin 1-$\beta$ (IL-1$\beta$);
Alpha-1-acid glycoprotein (A1AGP) and Matrix metalloproteinase-9 (MMP9).

The measurement device (A) should be able to receive a saliva sample, for example by putting a drop of saliva on a cartridge (A1), which can be inserted into the device (A). The device may be an existing device that is capable to determine, from the same saliva sample, the concentrations of at least two of the protein biomarkers of the invention, for example A1AGP and IL1-$\beta$, or MMP-8.

The processing unit (C) receives numerical values for the protein concentrations from part (A). The unit (C) is provided with software (typically embedded software) allowing it to calculate a score (S) between 0 and 1. The software further includes a numerical value for the threshold (T). If the calculated value (S) exceeds (T), unit (C) will output an indication (I) of 'successful treatment of periodontitis' to the GUI (B), otherwise it will output 'unsuccessful treatment of periodontitis'. A further embodiment may use the specific value of (S) to indicate the certainty with which the indication (I) is made. This can be 'directly', in the sense that e.g. a score S=0.8 would indicate 'successful treatment of periodontitis' with 80% certainty. Otherwise, it can be done e.g. through the definition of a range R1-R2, such that when R1<S<R2, the indication (I) will read 'inconclusive'.

A specific calculation of the scores can be implemented, e.g., by means of a logistic regression, employing the sigmoid function:

$$S = \frac{1}{1 + \exp(-(c_0 + \sum_{i=1}^{N} c_i B_i))}$$

With N the number of proteins/biomarkers used. $c_0$, $c_1$, etc. are coefficients (numerical values) and $B_1$, $B_2$, etc. are the respective protein concentrations.

Determination of the coefficients $c_i$ can be done by a training procedure:

- Select N1 subjects who have a successful response to treatment of periodontitis (as identified by a dentist via the current criteria) and N2 subjects who have a unsuccessful response to treatment of periodontitis.
- Take a saliva sample from each subject and determine the protein concentrations of a combination of biomarkers as explained above (the saliva sample may be from a patient that has not yet received a treatment [for predicting a response], or a patient that has received a treatment [for assessing the response])
- Define the score S to be 1 for successful response, and 0 for unsuccessful response.
- Fit the sigmoid function to the scores and protein concentration values.

Note that alternatively any existing regression or machine learning method (e.g. linear regression, neural networks, support vector machines, etc.) may be used, where the score S is high for patients successfully responding to treatment and low for the patients not successfully responding to treatment.

In particular, such a procedure has been applied using a clinical study with subjects who showed either successful response to treatment or unsuccessful response to treatment of periodontitis, which was identified by clinical assessment by a dental professional via below criteria:

| Classification | | Clinical condition, post-treatment status |
|---|---|---|
| Successful treatment response | **Success level** | |
| | 1 (best) | Probing depths: all sites ≤ 3 mm<br>BOP ≤ 10% |
| | 2 | Probing depths: all sites ≤ 4 mm<br>BOP ≤ 15% |
| | 3 | Probing depths: 90% of sites ≤ 4 mm, and no site > 5 mm<br>BOP ≤ 15% |
| | 4 | Probing depths: 90% of sites ≤ 5 mm, and no site > 6 mm<br>BOP ≤ 20% |
| Unsuccessful treatment | | None of the above conditions are satisfied |

Success level 1:

Indicates a "gold star" treatment outcome, probably relatively unlikely in most periodontitis patients but may be achieved in a small number of patients.

Success level 2:

Indicates a very good treatment response in a periodontitis patient.

Success level 3:

Indicates a pragmatic good treatment response, in that the vast majority of sites are ≤ 4 mm and no more than 10% of sites have probing depths of 5 mm. (And no sites have probing depths of $\geq$ 6 mm).

Success level 4:

Indicates a pragmatic moderately good treatment response, in that the vast majority of sites are $\leq$ 5 mm, and no more than 10% of sites have a probing depth of 6 mm. (And no sites have probing depths of $\geq$ 7 mm).

As used herein, "successful treatment" means any of Success levels 1 to 4. Accordingly the minimum clinical presentation deemed as "successful treatment" in a patient previously diagnosed with periodontitis is 90% of sites $\leq$ 5 mm probing depth, no site > 6 mm probing depth, and $\leq$ 20% Bleeding on Probing.

A multiclass classifier that indicates the "success" status may be used.

Performance of various biomarker combinations were evaluated by means of Leave-1-out cross validation, resulting in the preferred biomarker combinations of the invention.

With reference to the aforementioned system, the invention also provides, in a further aspect, a system for assessing whether a human patient has been or will be successfully treated for periodontitis, the system comprising:

- detection means able and adapted to detect in a sample of saliva of the human patient the proteins:

  (i) Alpha-1-acid glycoprotein (A1AGP) and at least one of Interleukin-1-beta (IL-1$\beta$) and Matrix metalloproteinase-8 (MMP-8); or
  (ii) Alpha-1-acid glycoprotein (A1AGP) and Interleukin-1-beta (IL-1$\beta$), and at least one of Matrix metalloproteinase-8 (MMP-8), Matrix metalloproteinase-9 (MMP-9) and Keratin-4 (K-4); or
  (iii) Alpha-1-acid glycoprotein (A1AGP) and Matrix metalloproteinase-9 (MMP-9).

As explained above, such means are known, and easily accessible to the skilled person. Typically, there is provided:

- a container for receiving an oral sample of a subject therein, the container provided with the detection means;
- a processor able and adapted to determine from the determined concentrations of said proteins an indication of yes/no successful response to treatment of periodontitis.

Optionally, the system comprises a user interface (or a data connection to remote interface), particularly a graphical user interface (GUI), capable of presenting information; a GUI is a type of user interface that allows users to interact with electronic devices through graphical icons and visual indicators such as secondary notation, instead of text-based user interfaces, typed command labels or text navigation (none of such interface types being excluded in the present invention); GUIs are generally known, and are used typically in handheld mobile devices such as MP3 players, portable media players, gaming devices, smartphones and smaller household, office and industrial controls; as said, the interface optionally can also be chosen so as to be capable of putting in information, such as, e.g., the age of the subject, sex, BMI (Body Mass Index).

The invention also provides, either separately or as part of the aforementioned system, a kit for detecting at least two biomarkers for periodontal disease in a sample of saliva of a human patient, said kit comprising detection reagents consisting of:

a first detection reagent for detecting A1AGP, a second detection reagent for detecting IL-1$\beta$, and a third detection reagent for detecting one of MMP-9, K-4 and MMP-8.

As discussed above with reference to the method of the invention, the kit may comprise more detection reagents, such as for Pyruvate Kinase and/or for other proteins. In a preferred embodiment the detection reagents made available in the kit consist of the detection reagents for the detection of three proteins making up a biomarker panel of the invention, as mentioned.

Preferably said kits comprise a solid support, such as a chip, a microtiter plate or a bead or resin comprising said detection reagents. In some embodiments, the kits comprise mass spectrometry probes, such as ProteinChip™.

The kits may also provide washing solutions and/or detection reagents specific for either unbound detection reagent or for said biomarkers (sandwich type assay).

In an interesting aspect, the recognition of a biomarker panel of the invention is applied in monitoring the status of periodontal disease in a human patient, over a period of treatment. Accordingly, the invention also provides an *in vitro* method for determining a change in status of periodontal disease due to treatment of the disease in a human patient suffering from periodontitis over a therapeutic time interval from a first time point $t_1$ to a second time point $t_2$, the method comprising detecting, in at least one sample of saliva obtained from said patient at $t_1$ and in at least one sample of saliva obtained from said patient at $t_2$, the concentrations of the proteins:

(i) Alpha-1-acid glycoprotein (A1AGP) and at least one of Interleukin-1-beta (IL-1β) and Matrix metalloproteinase-8 (MMP-8); or

(ii) Alpha-1-acid glycoprotein (A1AGP) and Interleukin-1-beta (IL-1β), and at least one of Matrix metalloproteinase-8 (MMP-8), Matrix metalloproteinase-9 (MMP-9) and Keratin-4 (K-4); or

(iii) Matrix metalloproteinase-9 (MMP-9) and Alpha-1-acid glycoprotein (A1AGP); and comparing the concentrations, whereby a difference of preferably two, three, four, or more concentrations, reflects a change in status. This difference can be reviewed as a difference in concentrations, thus allowing a direct comparison without first generating a number between 0 and 1, or any other classification. It will be understood that the measurements received at both points in time can also be processed in just the same manner as done when determining the patient status as above.

The invention also provides a method of diagnosing whether a human patient has been or will be successfully treated for periodontal disease, comprising detecting in the patient's saliva the presence of the proteins of the invention. The presence of successful therapy in the patient is assessed on the basis of the concentrations of said proteins in said sample. Optionally, the method of this aspect comprises the further step of treating the periodontal disease in the patient. This optional treatment step can comprise the administration of known therapeutic agents or dental procedures, or a combination of therapeutic agents and dental procedures. Known therapeutic agents include the administration of antimicrobial-containing agents such as a mouthwash, chip, gel or microsphere. A typical antimicrobial agent for use in treating gingivitis and periodontitis is chlorhexidine. Other therapeutic agents include antibiotics, typically orally-administered antibiotics, and enzyme suppressants such as doxycycline. Known non-surgical therapeutic procedures include scaling and root planing (SRP). Known surgical procedures include surgical pocket reduction, flap surgery, gum grafts or bone grafts.

The disclosure further provides a method of detecting the proteins of the invention in a patient, comprising:

(a) obtaining a saliva sample from a human patient; and

(b) detecting whether the proteins of the invention are present in the saliva sample by contacting the saliva sample with detection reagents for the proteins and detecting binding between each protein and detection reagent.

The invention will be further illustrated with reference to the following nonlimiting example.

**Example**

In a clinical study with 106 subjects undergoing repeated clinical visits at two independent clinical sites, who received treatment of periodontitis, of who:

- 74 had low/unsuccessful response
- 32 had high/successful response

we obtained Receiver-Operator-Characteristic Area-Under-the Curve values of >0.75 for detecting successful treatment outcome.

In statistics, a receiver operating characteristic curve, or ROC curve, is a graphical plot that illustrates the performance of a binary classifier system as its discrimination threshold is varied. The curve is created by plotting the true positive rate (TPR) against the false positive rate (FPR) at various threshold settings. The true-positive rate is also known as sensitivity, recall or *probability of detection* in machine learning. The false-positive rate is also known as the fall-out or *probability of false alarm* and can be calculated as (1 - specificity). The ROC curve is thus the sensitivity as a function of fall-out. In general, if the probability distributions for both detection and false alarm are known, the ROC curve can be generated by plotting for every value of the threshold, the value of the cumulative distribution function (area under the probability distribution from -∞ to the discrimination threshold) of the detection probability on the y-axis, versus the value of the cumulative distribution function of the false-alarm probability on the x-axis. The accuracy of the test depends on how well the test separates the group being tested into those with and without the disease in question. Accuracy is measured by the area under the ROC curve. An area of 1 represents a perfect test; an area of 0.5 represents a worthless test. A guide for classifying the accuracy of a diagnostic test is the traditional academic point system:

-

$$0.90\text{-}1 = \text{excellent (A)}$$

-

$$0.80\text{-}0.90 = \text{good (B)}$$

$$0.70\text{-}0.80 = \text{fair (C)}$$

$$0.60\text{-}0.70 = \text{poor (D)}$$

$$0.50\text{-}0.60 = \text{fail (F)}$$

Various biomarker combinations were evaluated by means of logistic regression with leave-one-out cross validation (LOOCV), resulting in the biomarker combinations of the invention. It can be seen that the protein biomarker combinations of the invention have an AUC of >0.75 for detecting successful treatment outcome.

Based on the foregoing, in the results of the aforementioned clinical study, an ROC AUC value of above 0.75 is considered to represent a desirable accuracy for providing a test in accordance with the invention.

The protein biomarkers explored were:

- MMP8

- MMP9

- IL-1β

- HGF

- Free Light Chain (FLC) κ (kappa)

- Free light chain (FLC) λ (lambda)

- A1AGP

- Hb-beta

- Hb-delta

- Keratin 4

- Profilin

- Pyruvate Kinase

- S100A8

- S100A9

Furthermore, in the employed logistic regression we considered as additional predictors $\kappa + \lambda$, $\kappa - \lambda$, $\kappa / \lambda$.

Additionally we included age as predictor.

This yields a total number of 4204 possible non-redundant panels, having at most 4 protein biomarkers (panel having only age is not considered). Non-redundant here means that a panel including e.g. $\kappa + \lambda$ and $\kappa - \lambda$ as predictors is not considered, as in the logistic regression it gives the same result as the corresponding panel including $\kappa$ and $\lambda$ as predictors.

Note that not restricting the number of protein markers in a panel, yields a number of 98302 possible non-redundant panels (panel having only age is not considered) given the predictors mentioned above.

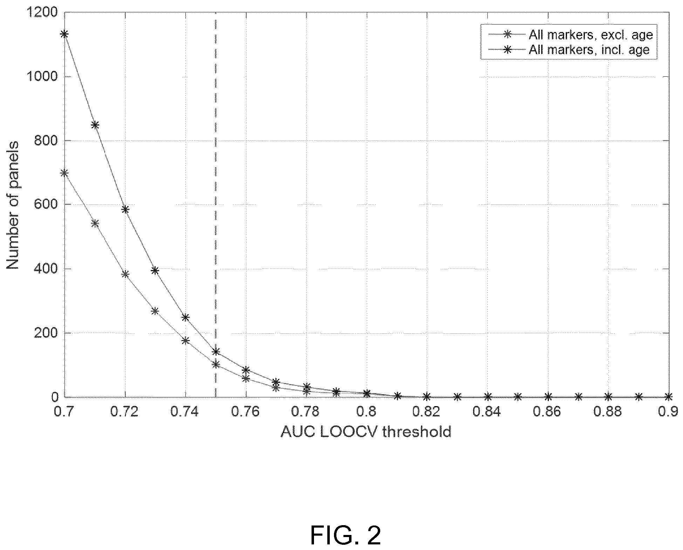

Fig. 2 displays the number of identified panels having at most 4 protein markers as function of the threshold in classification performance in terms of Receiver-Operator-Characteristic Area-Under-the-Curve when leave-one-out cross validation is employed (ROC AUC LOOCV). Separate graphs are shown for exclusion of age as predictor (lower [red] line with max 700 panels) as well as inclusion of age (upper [blue] line with max ~1150 panels).

At an AUC LOOCV threshold of 0.75, a number of 141 panels are found when including age, and 101 when excluding age.

For these panels at an AUC LOOCV threshold of 0.75, the prevalence of the different markers are indicated in below table:

| 101 Panels, excl. age as marker | | | 141 Panels, incl. age as marker | | | 40 Panels, explicitly with age | |
|---|---|---|---|---|---|---|---|
| MMP9 | 81 | | MMP9 | 109 | | Age | 40 |
| A1AGP | 56 | | IL1B | 89 | | IL1B | 34 |
| IL1B | 55 | | A1AGP | 84 | | A1AGP | 28 |
| Kappa | 28 | | Age | 40 | | MMP9 | 28 |
| HGF | 27 | | Kappa | 39 | | MMP8 | 13 |
| MMP8 | 24 | | MMP8 | 37 | | Kappa | 11 |
| S100A9 | 24 | | HGF | 34 | | HGF | 7 |
| Hb-beta | 23 | | Hb-beta | 29 | | Hb-beta | 6 |
| Hb-delta | 13 | | S100A9 | 29 | | Keratin 4 | 6 |
| Keratin 4 | 13 | | Keratin 4 | 19 | | S100A9 | 5 |
| Kappa/Lambda | 7 | | Hb-delta | 16 | | Hb-delta | 3 |
| Lambda | 6 | | Kappa/Lambda | 8 | | S100A8 | 3 |
| Kappa-Lambda | 5 | | Lambda | 8 | | Kappa+Lambda | 2 |
| Pyr. Kin. | 5 | | Pyr. Kin. | 7 | | Lambda | 2 |
| Profilin | 4 | | S100A8 | 7 | | Pyr. Kin. | 2 |
| S100A8 | 4 | | Kappa-Lambda | 6 | | Kappa-Lambda | 1 |
| Kappa+Lambda | 3 | | Kappa+Lambda | 5 | | Kappa/Lambda | 1 |
| Age | - | | Profilin | 5 | | Profilin | 1 |

Overall, these results show that MMP9, IL1B, A1AGP, Kappa, MMP8, and HGF are the most prevalent markers.

Preferred biomarker protein combinations that cover all 141 panels are:

- MMP9 & one or more of IL1B, HGF, A1AGP, HB-beta, S100A9
- A1AGP & one or more of MMP8, IL1B
- MMP8 & Kappa

(MMP9 & S100A9 covers one panel that also contains K/L)

The number of protein markers in these 141 panels are:

- One panel has two markers only: A1AGP & MMP9 (AUC LOOCV=0.751)

  - 20 panels have 3 markers

    - 120 panels have 4 markers

Preferred biomarker protein combinations that cover the 20 panels with 3 markers are:

- A1AGP and/or Kappa, and combination of 2 out of MMP8, MMP9, IL1B, HGF

- MMP9 & combination of 2 out of A1AGP, Kappa, IL1B, HB-beta, Hb-delta, Keratin 4, S100A9

The following 14 panels with ≤4 protein markers provide a performance of AUC LOOCV >0.8:

| MMP8 | IL1B | MMP9 | HGF | Age | κ | λ | κ+λ | κ/λ | κ-λ | A1AGP | Hb-beta | Hb-delta | Keratin 4 | Profilin | Pyruvate Kinase | S100A8 | S100A9 | AUC LOOCV |
|---|---|---|---|---|---|---|---|---|---|---|---|---|---|---|---|---|---|---|
| *Without age* | | | | | | | | | | | | | | | | | | |
|  | X | X |  |  |  |  |  |  |  | X |  |  |  |  |  |  |  | 0.815 |
| X | X | X |  |  |  |  |  |  |  | X |  |  |  |  |  |  |  | 0.801 |
|  | X | X | X |  |  |  |  |  |  | X |  |  |  |  |  |  |  | 0.803 |
|  | X | X |  | X |  |  |  |  |  | X |  |  |  |  |  |  |  | 0.818 |
|  | X | X |  |  |  | X |  |  |  | X |  |  |  |  |  |  |  | 0.807 |
|  | X | X |  |  |  |  |  |  |  | X | X |  |  |  |  |  |  | 0.814 |
|  | X | X |  |  |  |  |  |  |  | X |  | X |  |  |  |  |  | 0.809 |
| X | X |  |  |  |  |  |  |  |  | X |  |  | X |  |  |  |  | 0.801 |
|  | X | X |  |  |  |  |  |  |  | X |  |  | X |  |  |  |  | 0.810 |
|  | X | X |  |  |  |  |  |  |  | X |  |  |  |  | X |  |  | 0.801 |
|  | X | X |  |  |  |  |  |  |  | X |  |  |  |  |  |  | X | 0.804 |
| *With Age* | | | | | | | | | | | | | | | | | | |
| X | X |  | X |  |  |  |  |  |  | X |  |  |  |  |  |  |  | 0.801 |
| X | X |  | X | X |  |  |  |  |  | X |  |  |  |  |  |  |  | 0.809 |
| X | X |  | X |  |  |  |  |  |  | X | X |  |  |  |  |  |  | 0.803 |

Each of these combinations is a preferred biomarker combination according to the invention.

It can be seen that two panels have 3 protein markers and the rest have 4 protein markers. Protein biomarker combinations covering all of these 14 panels are:

- (IL1B & A1AGP) & one or both of MMP8* and MMP9

    *MMP8 may be replaced by Keratin 4

It is also noted that all but one panel is covered by IL1B & A1AGP & MMP9, which is therefore a preferred biomarker panel.

The identified panels above relate to measuring biomarker levels post-treatment in order to assess/estimate the likelihood of treatment success.

Also the feasibility of predicting treatment success from pre-treatment marker levels has been investigated. With the treatment success definition employed herein, it is found that a panel with **MMP8, IL1B, A1AGP, and optionally Pyruvate kinase** provides a performance of AUC LOOCV>0.75. This is therefore a preferred panel according to the invention, for predicting response to periodontitis treatment.

While the invention has been illustrated and described in detail in the drawings and foregoing description, such illustration and description are to be considered illustrative or exemplary and not restrictive; the invention is not limited to the disclosed embodiments. For example, it is possible to present detection reagents for different biomarkers in different units. Or, conveniently, a kit of the invention can comprise a fixed set of detection reagents for the protein biomarkers that are essential in an embodiments, i.e., A1AGP in certain embodiments, and flexible modules comprising a detection reagent for either of the further biomarkers, e.g. MMP-8 and/or IL-1β.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain features of the invention are recited in mutually different dependent claims does not indicate that a combination of these features cannot be used to advantage. Any reference signs in the claims should not be construed as limiting the scope.

In sum, we hereby disclose an *in vitro* method for assessing or predicting the response of a human patient to treatment of periodontal disease. The method is based on the insight to determine a selection of as few as two biomarker proteins. Accordingly, in a sample of saliva from a patient, the concentrations are measured of the proteins described herein. Based on the concentrations as measured, a value is determined reflecting the joint concentrations for said proteins. This value is typically used to calculate the probability of a treatment being successful, and compared with respective threshold values reflecting in the same manner the joint concentrations associated successful treatment of periodontitis. The comparison allows assessing whether the testing value is indicative of successful treatment of periodontitis in said patient.

## Claims

1. An *in vitro* method for assessing or predicting the response of a human patient to treatment of periodontal disease, wherein the method comprises:

   - detecting, in a sample of saliva from said human patient suffering from periodontal disease, the concentrations of the proteins:

      (i) Alpha-1-acid glycoprotein (A1AGP) and at least one of Interleukin-1-beta (IL-1β) and Matrix metalloproteinase-8 (MMP-8); or
      (ii) Alpha-1-acid glycoprotein (A1AGP) and Interleukin-1-beta (IL-1β), and at least one of Matrix metalloproteinase-8 (MMP-8), Matrix metalloproteinase-9 (MMP-9) and Keratin-4 (K-4); or
      (iii) Alpha-1-acid glycoprotein (A1AGP) and Matrix metalloproteinase-9 (MMP-9);

   - determining at least one testing value reflecting the joint concentrations determined for said proteins;
   - comparing said testing value with a threshold value reflecting in the same manner the joint concentrations associated with successful treatment of periodontal disease, so as to assess whether the testing value is indicative for successful treatment of periodontal disease in said patient.

2. An *in vitro* method according to claim 1, wherein:

   the method assesses the response of a human patient previously diagnosed as having periodontitis and that has received treatment for that periodontitis; or
   the method predicts the response of a human patient to a treatment for periodontitis, optionally wherein the treatment is proposed or was administered no more than 7, 6, 5, 4, 3, 2, or 1 days prior to assessment.

3. A method according to claim 1 or claim 2, wherein:

   the age of the subject is determined and the testing value reflects the joint concentrations determined for said proteins in combination with the age of the subject; and/or
   the threshold value is based on the concentration or concentrations determined for the proteins in one or more reference samples each sample associated with the successful treatment of periodontitis or the unsuccessful treatment of periodontitis; and/or
   the proteins consist of A1AGP, IL-1β, MMP-9, and K-4 or MMP-8.

4. A method according to any one of the preceding claims, wherein the concentration values determined are arithmetically processed into a number between 0 and 1.

5. The use of the proteins:

   (i) Alpha-1-acid glycoprotein (A1AGP) and at least one of Interleukin-1-beta (IL-1β) and Matrix metalloproteinase-8 (MMP-8); or
   (ii) Alpha-1-acid glycoprotein (A1AGP) and Interleukin-1-beta (IL-1β), and at least one of Matrix metalloproteinase-8 (MMP-8), Matrix metalloproteinase-9 (MMP-9) and Keratin-4 (K-4); or
   (iii) Alpha-1-acid glycoprotein (A1AGP) and Matrix metalloproteinase-9 (MMP-9);

   in a sample of saliva of a human patient, as biomarkers for assessing whether the patient will respond, or has responded to, periodontal disease treatment.

6. The use according to claim 5, wherein the age of the human patient is also used as a biomarker.

7. A system for assessing or predicting the response of a human patient to treatment of periodontal disease, the system comprising:

   - detection reagents to detect in a sample of saliva of the human patient the proteins:

     (i) Alpha-1-acid glycoprotein (A1AGP) and at least one of Interleukin-1-beta (IL-1β) and Matrix metalloproteinase-8 (MMP-8); or
     (ii) Alpha-1-acid glycoprotein (A1AGP) and Interleukin-1-beta (IL-1β), and at least one of Matrix metalloproteinase-8 (MMP-8), Matrix metalloproteinase-9 (MMP-9) and Keratin-4 (K-4); or
     (iii) Alpha-1-acid glycoprotein (A1AGP) and Matrix metalloproteinase-9 (MMP-9);

   - a processor adapted to perform the method of claim 1.

8. A system according to claim 7, further comprising a container for receiving an oral fluid sample, the container comprising the detection reagents.

9. A system according to claim 7 or 8, further comprising:

   - a user interface for presenting the indication to a user; and
   - a data connection between the processor and the user interface for transferring the indication from the processor to the user interface.

10. A system according to any one of claims 7 to 9, wherein:

    the processor is enabled to function by means of an internet-based application; and/or
    the interface is capable of putting in information on the age of the subject and the processor is able and adapted to determine from the determined concentrations of said proteins, an indication that the patient has been or will be successfully treated.

11. A kit for detecting three biomarkers for successful treatment of periodontal disease in a sample of saliva of a human patient, said kit comprising detection reagents consisting of a first detection reagent for detecting A1AGP, a second detecting reagent for detecting IL-1β, and a third detecting reagent for detecting one of MMP-9, K-4 and MMP-8, wherein a detection reagent is an agent or compound that specifically (or selectively) binds to, interacts with or detects the biomarker.

12. A kit according to claim 11, wherein the detection reagent includes an antibody, polyclonal antibody, or monoclonal antibody that preferentially binds the biomarker.

13. A kit according to claim 11 or 12, wherein the detection reagents are contained on a solid support.

14. An *in vitro* method for determining a change in status of periodontal disease due to treatment of the disease, in a human patient over a therapeutic time interval from a first time point $t_1$ to a second time point $t_2$, the method comprising detecting, in at least one sample of saliva obtained from said patient at $t_1$ and in at least one sample of saliva obtained from said patient at $t_2$, the concentrations of the proteins:

(i) Alpha-1-acid glycoprotein (A1AGP) and at least one of Interleukin-1-beta (IL-1β) and Matrix metalloproteinase-8 (MMP-8); or
(ii) Alpha-1-acid glycoprotein (A1AGP) and Interleukin-1-beta (IL-1β), and at least one of Matrix metalloproteinase-8 (MMP-8), Matrix metalloproteinase-9 (MMP-9) and Keratin-4 (K-4); or
(iii) Alpha-1-acid glycoprotein (A1AGP) and Matrix metalloproteinase-9 (MMP-9);

and comparing the concentrations, whereby a difference in any one, two, or three or more of the concentrations, reflects a change in status.

15. A method of determining whether a human patient has been or will be successfully treated for periodontal disease, comprising detecting in a sample of saliva of the human patient the proteins:

(i) Alpha-1-acid glycoprotein (A1AGP) and at least one of Interleukin-1-beta (IL-1β) and Matrix metalloproteinase-8 (MMP-8); or
(ii) Alpha-1-acid glycoprotein (A1AGP) and Interleukin-1-beta (IL-1β), and at least one of Matrix metalloproteinase-8 (MMP-8), Matrix metalloproteinase-9 (MMP-9) and Keratin-4 (K-4); or
(iii) Alpha-1-acid glycoprotein (A1AGP) and Matrix metalloproteinase-9 (MMP-9);

and assessing whether the human patient has been or will be successfully treated for periodontal disease on the basis of the concentrations of said proteins in said sample.

**Patentansprüche**

1. In-vitro-Verfahren zur Beurteilung oder zum Vorhersagen des Ansprechens eines menschlichen Patienten auf die Behandlung einer Parodontalerkrankung, wobei das Verfahren umfasst:

- Nachweisen der Konzentrationen der folgenden Proteine in einer Speichelprobe des an Parodontalerkrankung leidenden menschlichen Patienten:

(i) Alpha-1-Säure-Glykoprotein (A1AGP) und mindestens eines von Interleukin-1-beta (IL-1β) und Matrix-Metalloproteinase-8 (MMP-8); oder
(ii) Alpha-1-Säure-Glykoprotein (A1AGP) und Interleukin-1-beta (IL-1β) sowie mindestens eines von Matrix-Metalloproteinase-8 (MMP-8), Matrix-Metalloproteinase-9 (MMP-9) und Keratin-4 (K-4); oder
(iii) Alpha-1-Säure-Glykoprotein (A1AGP) und Matrix-Metalloproteinase-9 (MMP-9);

- Bestimmen mindestens eines Testwerts, der die gemeinsamen Konzentrationen, die für die Proteine bestimmt werden, widerspiegelt;
- Vergleichen des Testwerts mit einem Schwellenwert, der in gleicher Weise die mit einer erfolgreichen Behandlung der Parodontalerkrankung verbundenen gemeinsamen Konzentrationen widerspiegelt, um zu beurteilen, ob der Testwert für eine erfolgreiche Behandlung der Parodontalerkrankung bei dem Patienten indikativ ist.

2. In-Vitro-Verfahren nach Anspruch 1, wobei:

das Verfahren das Ansprechen eines menschlichen Patienten beurteilt, bei dem zuvor Parodontitis diagnostiziert wurde und der eine Behandlung gegen diese Parodontitis erhalten hat; oder
das Verfahren das Ansprechen eines menschlichen Patienten auf eine Behandlung gegen Parodontitis vorhersagt, wobei die Behandlung optional nicht mehr als 7, 6, 5, 4, 3, 2 Tage bzw. 1 Tag vor der Beurteilung vorgeschlagen wurde oder verabreicht wurde.

3. Verfahren nach Anspruch 1 oder Anspruch 2, wobei:

das Alter des Probanden bestimmt wird und der Testwert die gemeinsamen Konzentrationen, die für die Proteine bestimmt werden, in Kombination mit dem Alter des Probanden widerspiegelt; und/oder
der Schwellenwert auf der Konzentration oder den Konzentrationen basiert, die für die Proteine in einer oder mehreren Referenzproben bestimmt wurden, wobei jede Probe mit der erfolgreichen Behandlung der Parodontitis oder der nicht erfolgreichen Behandlung der Parodontitis verbunden ist; und/oder

die Proteine aus A1AGP, IL-1β, MMP-9 und K-4 oder MMP-8 bestehen.

4. Verfahren nach einem der vorstehenden Ansprüche, wobei die bestimmten Konzentrationswerte arithmetisch zu einer Zahl zwischen 0 und 1 verarbeitet werden.

5. Verwendung der Proteine:

(i) Alpha-1-Säure-Glykoprotein (A1AGP) und mindestens eines von Interleukin-1-beta (IL-1β) und Matrix-Metalloproteinase-8 (MMP-8); oder
(ii) Alpha-1-Säure-Glykoprotein (A1AGP) und Interleukin-1-beta (IL-1β) sowie mindestens eines von Matrix-Metalloproteinase-8 (MMP-8), Matrix-Metalloproteinase-9 (MMP-9) und Keratin-4 (K-4); oder
(iii) Alpha-1-Säure-Glykoprotein (A1AGP) und Matrix-Metalloproteinase-9 (MMP-9);

in einer Speichelprobe eines menschlichen Patienten als Biomarker zur Beurteilung, ob der Patient auf eine Behandlung von Parodontalerkrankung anspricht oder angesprochen hat.

6. Verwendung nach Anspruch 5, wobei das Alter des menschlichen Patienten auch als Biomarker verwendet wird.

7. System zur Beurteilung oder zum Vorhersagen des Ansprechens eines menschlichen Patienten auf die Behandlung einer Parodontalerkrankung, das System umfassend:

- Nachweisreagenzien zum Nachweisen der Proteine in einer Speichelprobe eines menschlichen Patienten:

(i) Alpha-1-Säure-Glykoprotein (A1AGP) und mindestens eines von Interleukin-1-beta (IL-1β) und Matrix-Metalloproteinase-8 (MMP-8); oder
(ii) Alpha-1-Säure-Glykoprotein (A1AGP) und Interleukin-1-beta (IL-1β) sowie mindestens eines von Matrix-Metalloproteinase-8 (MMP-8), Matrix-Metalloproteinase-9 (MMP-9) und Keratin-4 (K-4); oder
(iii) Alpha-1-Säure-Glykoprotein (A1AGP) und Matrix-Metalloproteinase-9 (MMP-9);

- ein Prozessor, der zur Durchführung des Verfahrens nach Anspruch 1 angepasst ist.

8. System nach Anspruch 7, weiter umfassend einen Behälter zum Aufnehmen einer Mundflüssigkeitsprobe, der Behälter umfassend die Nachweisreagenzien.

9. System nach Anspruch 7 oder 8, weiter umfassend:

- eine Benutzerschnittstelle zum Darstellen des Hinweises für einen Benutzer; und
- eine Datenverbindung zwischen dem Prozessor und der Benutzerschnittstelle zum Übertragen des Hinweises von dem Prozessor an die Benutzerschnittstelle.

10. System nach einem der Ansprüche 7 bis 9, wobei:

der Prozessor dazu befähigt ist, mittels einer internetbasierten Anwendung zu arbeiten; und/oder
die Schnittstelle in der Lage ist, Informationen über das Alter des Probanden einzugeben, und der Prozessor in der Lage ist und darauf ausgelegt ist, anhand der ermittelten Konzentrationen der genannten Proteine einen Hinweis darauf zu geben, dass der Patient erfolgreich behandelt wurde oder behandelt werden wird.

11. Kit zum Nachweisen von drei Biomarkern für die erfolgreiche Behandlung von Parodontalerkrankung in einer Speichelprobe eines menschlichen Patienten, wobei das Kit umfassend Nachweisreagenzien, bestehend aus einem ersten Nachweisreagenz zum Nachweisen von A1AGP, einem zweiten Nachweisreagenz zum Nachweis von IL-1β und einem dritten Nachweisreagenz zum Nachweis von MMP-9, K-4 und MMP-8, wobei ein Nachweisreagenz ein Mittel oder eine Verbindung ist, die spezifisch (oder selektiv) an den Biomarker bindet, mit ihm interagiert oder ihn nachweist.

12. Kit nach Anspruch 11, wobei die Nachweisreagenzien einen Antikörper, einen polyklonalen Antikörper oder einen monoklonalen Antikörper einschließen, der den Protein-Biomarker bevorzugt bindet.

13. Kit nach Anspruch 11 oder 12, wobei die Nachweisreagenzien auf einem festen Träger enthalten sind.

**14.** In-vitro-Verfahren zum Bestimmen einer Änderung im Status der Parodontalerkrankung bei einem menschlichen Patienten über einen Zeitraum von einem ersten Zeitpunkt $t_1$ bis zu einem zweiten Zeitpunkt $t_2$ bereit, das Verfahren umfassend Nachweisen, in mindestens einer Speichelprobe, die von dem Patienten bei $t_1$ erhalten wird, und in mindestens einer Speichelprobe, die von dem Patienten bei $t_2$ erhalten wird, der Konzentrationen der Proteine:

(i) Alpha-1-Säure-Glykoprotein (A1AGP) und mindestens eines von Interleukin-1-beta (IL-1β) und Matrix-Metalloproteinase-8 (MMP-8); oder

(ii) Alpha-1-Säure-Glykoprotein (A1AGP) und Interleukin-1-beta (IL-1β) sowie mindestens eines von Matrix-Metalloproteinase-8 (MMP-8), Matrix-Metalloproteinase-9 (MMP-9) und Keratin-4 (K-4); oder

(iii) Alpha-1-Säure-Glykoprotein (A1AGP) und Matrix-Metalloproteinase-9 (MMP-9);

und Vergleichen der Konzentrationen, wobei ein Unterschied in einer, zwei, drei oder mehreren Konzentrationen eine Statusänderung widerspiegelt.

**15.** Verfahren zum Bestimmen, ob ein menschlicher Patient erfolgreich gegen Parodontalerkrankung behandelt wurde oder wird, umfassend Nachweisen der folgenden Proteine in einer Speichelprobe des menschlichen Patienten:

(i) Alpha-1-Säure-Glykoprotein (A1AGP) und mindestens eines von Interleukin-1-beta (IL-1β) und Matrix-Metalloproteinase-8 (MMP-8); oder

(ii) Alpha-1-Säure-Glykoprotein (A1AGP) und Interleukin-1-beta (IL-1β) sowie mindestens eines von Matrix-Metalloproteinase-8 (MMP-8), Matrix-Metalloproteinase-9 (MMP-9) und Keratin-4 (K-4); oder

(iii) Alpha-1-Säure-Glykoprotein (A1AGP) und Matrix-Metalloproteinase-9 (MMP-9);

und Beurteilung, ob der menschliche Patient erfolgreich gegen Parodontalerkrankung behandelt wurde oder behandelt werden wird, basierend auf der Konzentrationen der Proteine in der Probe.

**Revendications**

**1.** Procédé *in vitro* pour évaluer ou prédire la réponse d'un patient humain au traitement d'une maladie parodontale, dans lequel le procédé comprend :

- la détection, dans un échantillon de salive dudit patient humain souffrant d'une maladie parodontale, des concentrations des protéines suivantes :

(i) la glycoprotéine alpha-1-acide (A1AGP) et au moins une de l'interleukine-1-bêta (IL-1β) et de la métalloprotéinase-8 matricielle (MMP-8) ; ou

(ii) la glycoprotéine alpha-1-acide (A1AGP) et l'interleukine-1-bêta (IL-1β), et au moins une de la métalloprotéinase-8 matricielle (MMP-8), de la métalloprotéinase-9 matricielle (MMP-9) et de la kératine-4 (K-4) ; ou

(iii) la glycoprotéine alpha-1-acide (A1AGP) et la métalloprotéinase-9 matricielle (MMP-9) ;

- la détermination d'au moins une valeur d'essai reflétant les concentrations communes déterminées pour lesdites protéines ;

- la comparaison de ladite valeur d'essai à une valeur seuil reflétant de la même manière les concentrations communes associées à un traitement réussi de maladie parodontale, de manière à évaluer si la valeur d'essai indique un traitement réussi de maladie parodontale chez ledit patient.

**2.** Procédé *in vitro* selon la revendication 1, dans lequel :

le procédé évalue la réponse d'un patient humain précédemment diagnostiqué comme souffrant de parodontite et qui a reçu un traitement pour cette parodontite ; ou

le procédé prédit la réponse d'un patient humain à un traitement contre la parodontite, facultativement dans lequel le traitement est proposé ou a été administré pas plus de 7, 6, 5, 4, 3, 2 ou 1 jours avant une évaluation.

**3.** Procédé selon la revendication 1 ou la revendication 2, dans lequel :

l'âge du sujet est déterminé et la valeur d'essai reflète les concentrations communes déterminées pour lesdites protéines en association avec l'âge du sujet ; et/ou

la valeur seuil est basée sur la concentration ou les concentrations déterminées pour les protéines dans un ou plusieurs échantillons de référence, chaque échantillon étant associé au traitement réussi de parodontite ou à l'échec de traitement de parodontite ; et/ou

les protéines se composent de l'A1AGP, de l'IL-1β, de la MMP-9 et de la K-4 ou de la MMP-8.

4. Procédé selon l'une quelconque des revendications précédentes, dans lequel les valeurs de concentration déterminées sont traitées arithmétiquement en un nombre compris entre 0 et 1.

5. Utilisation des protéines :

(i) la glycoprotéine alpha-1-acide (A1AGP) et au moins une de l'interleukine-1-bêta (IL-1β) et de la métalloprotéinase-8 matricielle (MMP-8) ; ou
(ii) la glycoprotéine alpha-1-acide (A1AGP) et l'interleukine-1-bêta (IL-1β), et au moins une de la métalloprotéinase-8 matricielle (MMP-8), de la métalloprotéinase-9 matricielle (MMP-9) et de la kératine-4 (K-4) ; ou
(iii) la glycoprotéine alpha-1-acide (A1AGP) et la métalloprotéinase-9 matricielle (MMP-9) ;

dans un échantillon de salive d'un patient humain, comme biomarqueurs pour évaluer si le patient répondra, ou a répondu, à un traitement de maladie parodontale.

6. Utilisation selon la revendication 5, dans laquelle l'âge du patient humain est également utilisé comme biomarqueur.

7. Système pour évaluer ou prédire la réponse d'un patient humain à un traitement de maladie parodontale, le système comprenant :

- des réactifs de détection pour détecter, dans un échantillon de salive du patient humain, les protéines suivantes :

(i) la glycoprotéine alpha-1-acide (A1AGP) et au moins une de l'interleukine-1-bêta (IL-1β) et de la métalloprotéinase-8 matricielle (MMP-8) ; ou
(ii) la glycoprotéine alpha-1-acide (A1AGP) et l'interleukine-1-bêta (IL-1β), et au moins une de la métalloprotéinase-8 matricielle (MMP-8), de la métalloprotéinase-9 matricielle (MMP-9) et de la kératine-4 (K-4) ; ou
(iii) la glycoprotéine alpha-1-acide (A1AGP) et la métalloprotéinase-9 matricielle (MMP-9) ;

- un processeur adapté pour réaliser le procédé selon la revendication 1.

8. Système selon la revendication 7, comprenant en outre un récipient destiné à recevoir un échantillon de liquide buccal, le récipient comprenant les réactifs de détection.

9. Système selon la revendication 7 ou 8, comprenant en outre :

- une interface utilisateur pour présenter l'indication à un utilisateur ; et
- une connexion de données entre le processeur et l'interface utilisateur pour transférer l'indication du processeur à l'interface utilisateur.

10. Système selon l'une quelconque des revendications 7 à 9, dans lequel :

le processeur est autorisé à fonctionner au moyen d'une application basée sur Internet ; et/ou
dans lequel l'interface est capable d'introduire des informations sur l'âge du sujet et le processeur est capable et adapté pour déterminer, à partir des concentrations déterminées desdites protéines, une indication permettant de savoir si le patient a été, ou sera, traité avec succès.

11. Kit de détection de trois biomarqueurs pour un traitement réussi de maladie parodontale dans un échantillon de salive d'un patient humain, ledit kit comprenant des réactifs de détection composés d'un premier réactif de détection pour détecter l'A1AGP, d'un deuxième réactif de détection pour détecter l'IL-1β et d'un troisième réactif de détection pour détecter l'une de la MMP-9, de la K-4 et de la MMP-8, dans lequel un réactif de détection est un agent ou un composé qui se lie spécifiquement (ou sélectivement) au biomarqueur, interagit avec lui ou le détecte.

12. Kit selon la revendication 11, dans lequel le réactif de détection inclut un anticorps, un anticorps polyclonal ou un anticorps monoclonal qui se lie préférentiellement au biomarqueur.

**13.** Kit selon la revendication 11 ou 12, dans lequel les réactifs de détection sont contenus sur un support solide.

**14.** Procédé *in vitro* pour déterminer un changement d'état de maladie parodontale sous l'effet d'un traitement de la maladie chez un patient humain sur un intervalle de temps de thérapie allant d'un premier instant $t_1$ à un second instant $t_2$, le procédé comprenant la détection, dans au moins un échantillon de salive obtenu dudit patient à $t_1$ et dans au moins un échantillon de salive obtenu dudit patient à $t_2$, des concentrations des protéines suivantes :

(i) la glycoprotéine alpha-1-acide (A1AGP) et au moins une de l'interleukine-1-bêta (IL-1$\beta$) et de la métallo-protéinase-8 matricielle (MMP-8) ; ou
(ii) la glycoprotéine alpha-1-acide (A1AGP) et l'interleukine-1-bêta (IL-1$\beta$), et au moins une de la métalloprotéinase-8 matricielle (MMP-8), de la métalloprotéinase-9 matricielle (MMP-9) et de la kératine-4 (K-4) ; ou
(iii) la glycoprotéine alpha-1-acide (A1AGP) et la métalloprotéinase-9 matricielle (MMP-9) ;

et la comparaison des concentrations, selon lequel une différence d'une, de deux ou de trois, ou plus, concentrations reflète un changement d'état.

**15.** Procédé permettant de déterminer si un patient humain a été, ou sera, traité avec succès pour une maladie parodontale, comprenant la détection, dans un échantillon de salive du patient humain, des protéines suivantes :

(i) la glycoprotéine alpha-1-acide (A1AGP) et au moins une de l'interleukine-1-bêta (IL-1$\beta$) et de la métallo-protéinase-8 matricielle (MMP-8) ; ou
(ii) la glycoprotéine alpha-1-acide (A1AGP) et l'interleukine-1-bêta (IL-1$\beta$), et au moins une de la métalloprotéinase-8 matricielle (MMP-8), de la métalloprotéinase-9 matricielle (MMP-9) et de la kératine-4 (K-4) ; ou
(iii) la glycoprotéine alpha-1-acide (A1AGP) et la métalloprotéinase-9 matricielle (MMP-9) ;

et l'évaluation si le patient humain a été, ou sera, traité avec succès pour une maladie parodontale sur la base des concentrations desdites protéines dans ledit échantillon.

FIG. 1

FIG. 2

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RAMSEIER et al.** *J Periodontol.*, March 2009, vol. 80 (3), 436-46

- **W.M SEXTON**. Salivary biomarkers of periodontal disease in response to treatment. *Journal of Clinical Periodontology*, 2011, vol. 38, 434-441